# FASCICULE DE BREVET EUROPEEN

(11) **EP 3 174 598 B1**
(45) Date de publication et mention de la délivrance du brevet: **07.11.2018**
(21) Numéro de dépôt: 15738720.0
(22) Date de dépôt: 17.06.2015
(51) Int. Cl.: A61N 5/06, A61N 1/20, A61B 5/0476, A61B 5/04, A61B 5/0484, A61B 5/00

(54) **ÉQUIPEMENT POUR L'APPLICATION D'ÉLÉMENTS ACTIFS SUR LE CRÂNE D'UN PATIENT**
VORRICHTUNG ZUM ANBRINGEN VON AKTIVEN ELEMENTEN AM SCHÄDEL EINES PATIENTEN
EQUIPMENT FOR APPLYING ACTIVE ELEMENTS TO THE SKULL OF A PATIENT

(30) Priorité: 30.07.2014 FR 1457365
(43) Date de publication de la demande: 07.06.2017
(73) Titulaire: Jean-Tien, 35250 Mouaze (FR)
(72) Inventeur: NGUYEN, Jean, F-35250 Mouaze (FR)
(74) Mandataire: IP Trust
(86) Numéro de dépôt international: PCT/FR2015/051602
(87) Numéro de publication internationale: WO 2016/016526

(56) Documents cités:
- WO-A1-2005/058157
- WO-A1-2008/067839
- WO-A1-2012/130958
- WO-A2-96/36396
- WO-A2-2009/132855
- DE-A1-102012 110 358
- US-A1- 2010 204 762

## Description

### Domaine de l'invention

La présente invention concerne le domaine de de la stimulation non invasive du cerveau et des procédés et équipements pour l'application de traitements issus des connaissances en médecine moderne et médecine énergétique.

Elle concerne plus précisément les traitements par des actions exercées au niveau de la boîte crânienne, et plus particulièrement les équipements physiques pour la mise en oeuvre de tels traitements.

### Etat de la technique

On connaît dans l'état de la technique différentes solutions pour la réalisation de tels traitements.

A titre d'exemple, la demande de brevet américaine US 20140058189 décrit un système et un procédé de stimulation du cerveau pour le traitement de disfonctionnements cérébraux. Ce brevet décrit un dispositif de stimulation non invasive du cerveau, mettant en oeuvre des générateurs de champs électromagnétiques dont les paramètres sont ajustés dynamiquement en fonction de l'activité électro-encéphalographique.

Le brevet américain US6537304 décrit un appareil permettant de traiter une région ischémique de cellules cérébrales du crâne, qui comporte une calotte adaptée pour couvrir au moins une partie du crâne, au moins un dispositif de guidage attaché à la calotte, et une source laser servant à envoyer un rayon laser par le dispositif de guidage dans le crâne. Ce dispositif de guidage peut comporter une fibre optique ou un guide d'ondes.

Le brevet FR2159851 décrit un exemple de casque porte-électrodes pour le positionnement automatique d'électrodes d'EEG sur la tête d'un patient.

Le brevet européen EP2489405 décrit un dispositif portable destiné à produire une photothérapie pour le cerveau. Il comprend un casque couvrant une partie du crane du patient. Des sources lumineuses sont positionnées de manière amovible sur ce casque.

La demande de brevet américain US2010204762 ou le brevet européen EP2533858 décrivent un autre exemple de dispositif pour le positionnement d'éléments de photothérapie du cerveau.

Ces brevets concerne un appareil et un procédé destinés à indiquer les emplacements des sites de traitement par photothérapie sur le cerveau. Dans certains modes de réalisation, l'appareil est un casque pouvant être porté par un patient. Le casque comprend un corps conçu pour être porté sur au moins une partie du cuir chevelu du patient et une pluralité d'indicateurs de positions correspondant à une pluralité d'emplacements de sites de traitement sur le cuir chevelu du patient où une source lumineuse doit être positionnée de manière séquentielle de sorte que la lumière provenant de la source lumineuse soit appliquée de manière séquentielle afin d'irradier au moins une partie du cerveau.

Le brevet américain US8644940 décrit un système de stimulation cérébrale thérapeutique comprenant au moins deux émetteurs de signaux de stimulation qui produisent des signaux de stimulation depuis différentes positions en direction d'une région cible commune dans le cerveau d'un patient. Alors que l'intensité de chaque signal de stimulation est bien trop faible pour provoquer une stimulation, les signaux de stimulation cumulés engendrent une stimulation et ont par conséquent un effet thérapeutique dans les cellules neuronales du cerveau de la région cible. Les signaux de stimulation se cumulant dans la région cible peuvent être ajustés de manière à ne pas affecter négativement la structure anatomique des cellules neuronales du cerveau dans la région cible.

La demande de brevet EP2101640 concerne une casquette d'électrodes destinée à une électro-encéphalographie qui permet d'entrer en contact avec la tête d'une personne ou d'un animal sans gel conducteur entre le cuir chevelu et les électrodes elles-mêmes. Cette casquette d'électrodes est destinée à venir en contact avec le cuir chevelu de la tête dans une électro-encéphalographie avec un certain nombre d'électrodes en forme de broche destinées à venir en contact avec le cuir chevelu, et un organe de tenue d'électrode caractérisé en ce que les électrodes sont montées sur cet organe de tenue d'électrode via au moins une articulation élastique.

Le brevet EP1663392 concerne un appareil de traitement destiné à traiter le cerveau d'un patient. Cet appareil de traitement comprend un générateur de lumière possédant une zone d'émission de sortie disposée de sorte à irradier une partie du cerveau avec une densité de puissance et une longueur d'onde de lumière efficaces. Ledit appareil de traitement comprend en outre un élément intercalé entre le générateur de lumière et le cuir chevelu du patient.

Le brevet EP1429709 concerne un dispositif d'application d'au moins une aiguille laser sur le corps d'un patient. Ledit dispositif comporte un élément de fixation pouvant être adapté à une partie du corps du patient. Ledit élément de fixation présente au moins un guide pouvant recevoir l'aiguille laser de manière que celle-ci puisse être déplacée par rapport à la partie du corps au moyen dudit guide.

La demande de brevet EP2496135 concerne un dispositif permettant de positionner des électrodes sur le cuir chevelu d'un utilisateur. Le dispositif comprend un boîtier, par exemple un casque de dispositif d'écouteurs, qui peut être mis en place sur la tête d'un utilisateur. Un élément élastique et une pluralité d'électrodes sont positionnés, de sorte qu'une fois que le boîtier est mis en place sur la tête de l'utilisateur, l'élément élastique suit au moins partiellement la courbure de la tête de l'utilisateur. La contrainte de l'élément élastique due à l'étirement qu'il subit lors de la mise en place dudit boîtier sur la tête de l'utilisateur fait en sorte que l'élément élastique exerce une pression sur au moins certaines de la pluralité des électrodes, en direction du cuir chevelu.

Avantageusement, les éléments actifs sont des capteurs permettant de mesurer l'activité cérébrale ou une combinaison de capteurs et d'émetteurs permettant de mesurer l'activité cérébrale et de stimuler le cerveau simultanément.

### Inconvénients de l'art antérieur

Les solutions de l'art antérieur aboutissent à des positionnements erratiques des éléments actifs, et à des traitements empiriques et mal maitrisés.

### Solution apportée par l'invention

La présente invention vise à remédier à ces inconvénients en proposant un équipement pour l'application d'éléments actifs sur le crâne d'un patient, comprenant un support et une pluralité d'éléments actifs caractérisés en ce que ledit support comporte un moyen de liaison avec un patient et des moyens pour le positionnement desdits éléments actifs pour exciter N+M zones de la boîte crânienne, lesdites N zones comprenant les zones [VG₁₉, VG₂₀, VG₂₁, C₄, C_{5R}, C_{5L}, C_{6R} et C_{6L}], N et M étant deux nombres entiers, N étant un nombre compris entre 2 et 8, M étant inférieur à N/2.

Avantageusement, N est un nombre compris entre 4 et 8 et M est égal à 0.

De préférence, lesdits éléments actifs sont des lasers de faible puissance. On entendra par « faible puissance » au sens du présent brevet une puissance électrique inférieur à 60 mW.

Avantageusement, ledit laser présente une puissance comprise entre 1 et 15 mW.

De préférence, chacun des éléments actifs est constitué par un laser émettant un faisceau rouge, d'une longueur d'onde d'environ 650 nm.

Selon une variante, lesdits éléments actifs sont constitués par des diodes électroluminescentes (LED).

Selon une variante, lesdits éléments actifs sont constitués par des générateurs de champs électromagnétiques.

De préférence, l'équipement selon l'invention comporte un moyen de réglage de l'énergie appliqué à chacun desdits éléments actifs.

### Description détaillée d'un exemple non limitatif de réalisation

La présente invention sera mieux comprise à la lecture de la description qui suit, se référant à un exemple non limitatif de réalisation, où :
- la figure 1 représente une vue schématique d'un équipement selon l'invention
- la figure 2 représente une vue schématique de face de l'emplacement des zones de positionnement des éléments actifs

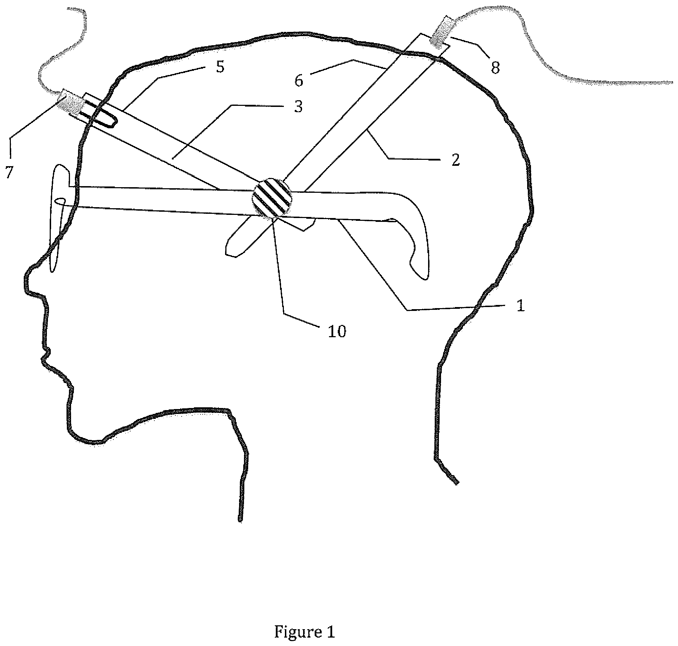

La figure 1 représente une vue schématique d'un équipement selon l'invention.

Il est constitué par une monture, présentant un support central ou frontal de la boite crânienne avec des extensions latérales (1). Cette monture constitue un référentiel pour le positionnement des capteurs en regard de certaines zones de la boîte crânienne.

Les extensions latérales de la monture (1) supportent au niveau de la zone temporale de la boite crânienne une pluralité d'arceaux (2, 3) en forme de serre-tête, articulés sur chaque extension latérale (1) par un pivot (10) permettant le réglage angulaire par rapport au référentiel crânien défini par la monture, puis le blocage de l'orientation relatives des arceaux (2, 3).

Ces arceaux (2, 3) présentent des lumières (5, 6) permettant le déplacement, sur les trajectoires définies par les arceaux (2, 3), d'éléments actifs (7, 8). Ces éléments actifs sont par exemple des diodes laser émettant dans le rouge (650 nanomètres) avec une puissance de 10 mW. Elles sont alimentées par un circuit électronique délivrant pour chacune des diodes un signal modulé avec une tension-crête de 3 volts.

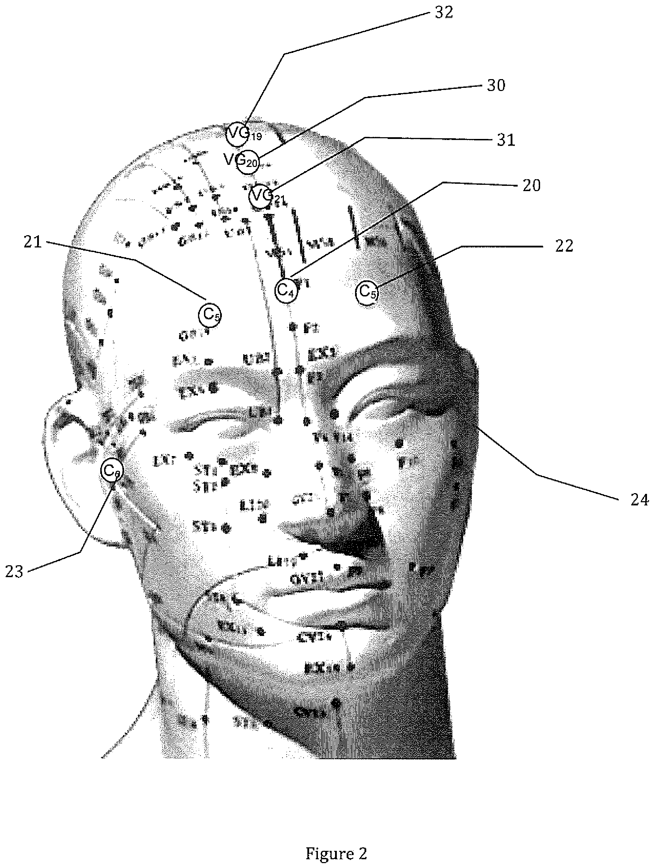

La figure 2 représente une vue schématique des zones d'actions des éléments actifs positionnés par un dispositif, par exemple celui décrit en figure 1.

Ces zones d'action sont choisies principalement parmi les zones suivantes :
A) Les zones (30 à 32) C₁, C₂ et C₃ situées sur le méridien du vaisseau du gouverneur.
   Elles comprennent le 20ème point du vaisseau gouverneur/Du Mai (VG₂₀). Cette zone VG₂₀ (30) est située à environ cinq centimètres en arrière de la ligne d'implantation des cheveux du front et à environ sept centimètres au-dessus de la ligne d'implantation des cheveux de la nuque, à l'intersection de la ligne médiane du crane et d'une ligne reliant le bout des deux oreilles (l'apex), sur l'aponévrose épicrânienne et entre les deux trous pariétaux.
   Le point VG₁₉ (ou Chakra 7) (32) est situé au sommet de la tête. Le point VG₂₁ (31) est situé sur la ligne médiane, symétriquement opposé au point (32) par rapport au point (30).
B) les quatre zones C₄, C₅ et C₆ appartenant aux points hors-méridiens de la face.

Le point C₄ (20) est situé au milieu du front. Les deux points C₅ (21, 22) sont situés de part et d'autre du point C₄ (20), sur la ligne horizontal passant par ce point C₄, à la verticale du milieu de chaque sourcil.

Les deux points C₆ (23, 24) sont situés sur les méridiens du triple réchauffeur, au niveau de la tempe.

La mise en oeuvre de l'appareil se fait par un positionnement de 4 à 8 actionneurs sur le support, pour correspondre à des points principalement choisis parmi les 8 points susvisés. Le choix des points susvisés est défini par le praticien en médecine pour un traitement thérapeutique précis.

Lorsque le patient est équipé, on alimente chacune des diodes avec une fréquence comprise entre 4 et 10 Hz, pendant une durée de 10 à 30 minutes.

### Alternative de réalisation

L'équipement peut être réalisé sous de nombreuses alternatives. En particulier, il peut prendre la forme d'un oreiller de soutien pour la tête et le cou comportant un élément de support de structure, formant un appui pour la nuque. Les éléments actifs sont fixés sur l'élément de support de structure, ainsi que sur des extensions supportant les éléments actifs pour les zones qui ne sont pas en contact avec la surface de l'oreiller.

## Revendications

1. Equipement pour l'application d'éléments actifs sur le crâne d'un patient, comprenant un support et une pluralité d'éléments actifs **caractérisé en ce que** ledit support comporte un moyen de liaison (1) avec un patient et des moyens du positionnement qui positionnent desdits éléments actifs au niveau de N+M zones (20 à 24 ; 30 à 32) de la boîte crânienne, lesdites N zones choisies parmi les zones VG₁₉, VG₂₀ et VG₂₁, situées sur le méridien du vaisseau du gouverneur, et les zones C₄, C_{5R}, C_{5L}, C_{6R} et C_{6L}, appartenant aux points hors-méridiens de la face, N et M étant deux nombres entiers, N étant un nombre compris entre 2 et 8, M étant inférieur à N/2.

2. Equipement pour l'application d'éléments actifs sur le crâne d'un patient selon la revendication 1 **caractérisé en ce que** N est un nombre compris entre 4 et 8 et M est égal à 0.

3. Equipement pour l'application d'éléments actifs sur le crâne d'un patient selon la revendication 1 **caractérisé en ce que** lesdits éléments actifs sont des diodes laser de faible puissance.

4. Equipement pour l'application d'éléments actifs sur le crâne d'un patient selon la revendication 3 **caractérisé en ce que** ledit laser présente une puissance comprise entre 1 et 15 mW.

5. Equipement pour l'application d'éléments actifs sur le crâne d'un patient selon la revendication 3 **caractérisé en ce que** ledit laser émet un faisceau dans le rouge.

6. Equipement pour l'application d'éléments actifs sur le crâne d'un patient selon la revendication 1 **caractérisé en ce que** lesdits éléments actifs sont constitués par des générateurs de champs électromagnétique.

7. Equipement pour l'application d'éléments actifs sur le crâne d'un patient selon la revendication 1 **caractérisé en ce qu'**il comporte un moyen de réglage de l'énergie appliqué à chacun desdits éléments actifs.

8. Equipement pour l'application d'éléments actifs sur le crâne d'un patient selon l'une quelconque des revendications précédentes **caractérisé en ce qu'**il comprend :
Un support central ou frontal de la boite crânienne avec des extensions latérales qui constitue un référentiel pour le positionnement d'éléments actifs en regard de zones précises de la boîte crânienne.
Une ou plusieurs extension latérales qui supportent au niveau de la zone temporale de la boite crânienne une pluralité d'arceaux articulés sur chaque extension latérale (1) par un pivot (10) permettant le réglage angulaire par rapport au référentiel crânien défini par la monture, puis le blocage de l'orientation relatives des arceaux (2, 3).
Des arceaux qui présentent des lumières permettant le déplacement, sur les trajectoires définies par les arceaux des éléments actifs

9. Equipement pour l'application d'éléments actifs sur le crâne d'un patient selon l'une quelconque des revendications précédentes **caractérisé en ce qu'**il comprend un oreiller de soutien pour la tête et le cou comportant un élément de support de structure, formant un appui pour la nuque, les éléments actifs étant fixés sur l'élément de support de structure, ainsi que sur des extensions supportant les éléments actifs pour les zones qui ne sont pas en contact avec la surface de l'oreiller

10. Equipment selon les revendications 1, 8 ou 9 pour lequel les éléments actifs sont des capteurs permettant de mesurer l'activité cérébrale

11. Equipment selon les revendications 1, 8 ou 9 pour lequel les éléments actifs sont des émetteurs ou sources d'énergie permettant de stimuler le cerveau

12. Equipement selon les revendications 1, 8 ou 9 pour lequel les éléments actifs sont une combinaison de capteurs et d'émetteurs permettant de mesurer l'activité cérébrale et de stimuler le cerveau simultanément

## Patentansprüche

1. Vorrichtung zur Anwendung aktiver Elemente auf den Schädel eines Patienten, welche eine Halterung und eine Vielzahl aktiver Elemente enthält, **dadurch gekennzeichnet, dass** besagte Halterung Mittel (1) zur Herstellung einer Verbindung mit einem Patienten umfasst, sowie Positionierungsmittel, die besagte aktive Elemente in N+M Zonen (20 bis 24; 30 bis 32) der Schädelhöhle positionieren, wobei N Zonen aus den Zonen VG₁₉, VG₂₀ und VG₂₂ auf dem Lenkermeridian und den Zonen C₄, C_{5R}, C_{5L}, C_{6R} und C_{6L} der Extrapunkte des Gesichtsmeridians ausgewählt werden, wobei N und M zwei ganze Zahlen sind, N eine Zahl zwischen 2 und 8 und M kleiner N/2 ist.

2. Vorrichtung zur Anwendung aktiver Elemente auf den Schädel eines Patienten nach Anspruch 1, **dadurch gekennzeichnet, dass** N eine Zahl zwischen 4 und 8, und M gleich null ist.

3. Vorrichtung zur Anwendung aktiver Elemente auf den Schädel eines Patienten nach Anspruch 1, **dadurch gekennzeichnet, dass** besagte aktive Elemente Laserdioden mit geringer Leistung sind.

4. Vorrichtung zur Anwendung aktiver Elemente auf den Schädel eines Patienten nach Anspruch 3, **dadurch gekennzeichnet, dass** besagter Laser eine Leistung zwischen 1 und 15 MW aufweist.

5. Vorrichtung zur Anwendung aktiver Elemente auf dem Schädel eines Patienten nach Anspruch 3, **dadurch gekennzeichnet, dass** besagter Laser einen Strahl im roten Bereich emittiert.

6. Vorrichtung zur Anwendung aktiver Elemente auf den Schädel eines Patienten nach Anspruch 1, **dadurch gekennzeichnet, dass** besagte aktive Elemente aus Elektromagnetfeldgeneratoren bestehen.

7. Vorrichtung zur Anwendung aktiver Elemente auf den Schädel eines Patienten nach Anspruch 1, **dadurch gekennzeichnet, dass** sie ein Mittel zur Einstellung der auf jedes dieser aktiven Elemente angewandten Energie umfasst.

8. Vorrichtung zur Anwendung aktiver Elemente auf den Schädel eines Patienten nach einem der vorhergehenden Ansprüche, **dadurch gekennzeichnet, dass** sie folgendes umfasst:
eine mittige oder frontale Halterung für die Schädelhöhle mit seitlichen Erweiterungen, die ein Bezugssystem zur Positionierung aktiver Elemente gegenüber bestimmter Zonen der Schädelhöhle darstellt;
eine oder mehrere seitliche Erweiterungen, die im Bereich der Schläfenzone des Schädels eine Vielzahl an Bügeln stützen, die auf jeder seitlichen Erweiterung (1) durch einen Zapfen (10) angelenkt sind, der wiederum die Winkeleinstellung relativ zu dem über das Gestell definierten Bezugssystem des Schädels und die Blockierung der Ausrichtung in Bezug auf die Bügel (2, 3) ermöglicht.
Bügel, die Öffnungen darstellen, welche das Verschieben der aktiven Elemente auf Bahnen ermöglichen, die durch die Bügel abgegrenzt werden.

9. Vorrichtung zur Anwendung aktiver Elemente auf dem Schädel eines Patienten nach einem der vorhergehenden Ansprüche, **dadurch gekennzeichnet, dass** sie ein Kissen zum Stützen das Kopfs und des Halses mit einem Strukturträgerelement umfasst, das eine Auflage für den Nacken bildet, wobei die aktiven Elemente auf dem Strukturträgerelement sowie auf den Erweiterungen, welche die aktiven Elemente für Zonen stützen, die nicht mit der Oberfläche des Kissens in Berührung kommen, fixiert werden.

10. Vorrichtung nach den Ansprüchen 1, 8 oder 9, bei denen die aktiven Elemente Sensoren sind, die das Messen der Gehirntätigkeit ermöglichen.

11. Vorrichtung nach Anspruch 1, 8 oder 9, bei der die aktiven Elemente Sender oder Energiequellen sind, die die Stimulierung des Gehirns ermöglichen.

12. Vorrichtung nach den Ansprüchen 1, 8 oder 9, in denen die aktiven Elemente eine Kombination von Sensoren und Sendern sind, die das Messen der Gehirntätigkeit und die gleichzeitige Stimulierung des Gehirns ermöglichen.

## Claims

1. Equipment for applying active elements to the cranium of a patient, comprising a support and a plurality of active elements, **characterised in that** said support comprises a means (1) of connection to the patient and positioning means that positions said active elements at N+M zones (20 to 24; 30 to 32) of the skull, said N zones chosen from the zones VG₁₉, VG₂₀ and VG₂₁, situated on the meridian of the governing vessel, and the zones C₄, C_{SR}, C_{5L}, C_{6R} and C_{6L}, belonging to the off-meridian points of the face, N and M being two integers, N being a number between 2 and 8, M being less than N/2.

2. Equipment for applying active elements to the cranium of a patient according to claim 1, **characterised in that** N is a number between 4 and 8 and M is equal to 0.

3. Equipment for applying active elements to the cranium of a patient according to claim 1, **characterised in that** said active elements are low-power laser diodes.

4. Equipment for applying active elements to the cranium of a patient according to claim 3, **characterised in that** said laser has a power of between 1 and 15 mW.

5. Equipment for applying active elements to the cranium of a patient according to claim 3, **characterised in that** said laser emits a beam in the red range.

6. Equipment for applying active elements to the cranium of a patient according to claim 1, **characterised in that** said active elements consist of electromagnetic-field generators.

7. Equipment for applying active elements to the cranium of a patient according to claim 1, **characterised in that** it comprises a means for adjusting the energy applied to each of said active elements.

8. Equipment for applying active elements to the cranium of a patient according to any of the preceding claims, **characterised in that** it comprises:
a central or frontal support for the skull with lateral extensions that constitute a reference frame for the positioning of active elements opposite precise zones of the skull,
one or more lateral extensions that support, at the temporal zone of the skull, a plurality of arches articulated on each lateral extension (1) by a pivot (10) allowing angular adjustment with respect to the cranial reference frame defined by the mount, and then the locking of the relative orientations of the arches (2, 3),
arches that have apertures allowing movement on paths defined by the arches of the active elements.

9. Equipment for applying active elements to the cranium of a patient according to any of the preceding claims, **characterised in that** it comprises a support pillow for the head and neck comprising a structural support element, forming a support for the nape, the active elements being fixed to the structural support element, as well as to extensions supporting the active elements for the zones that are not in contact with the surface of the pillow.

10. Equipment according to claims 1, 8 or 9, for which the active elements are sensors for measuring brain activity.

11. Equipment according to claims 1, 8 or 9, for which the active elements are energy emitters or sources for stimulating the brain.

12. Equipment according to claims 1, 8 or 9, for which the active elements are a combination of sensors and emitters for measuring the brain activity and stimulating the brain simultaneously.
